# EUROPEAN PATENT APPLICATION

(11) **EP 4 545 016 A1**
(43) Date of publication of application: **30.04.2025**
(21) Application number: 23827169.6
(22) Date of filing: 19.06.2023
(51) Int. Cl.: A61B 17/12

(54) **EMBOLIC DEVICE**

(30) Priority: 22.06.2022 JP 2022100663
(71) Applicant: KANEKA CORPORATION, Osaka 530-8288 (JP)
(72) Inventor: OGAWA, Atsushi, Tokyo 107-6028 (JP); TAKATERA, Masayuki, Settsu-shi, Osaka 566-0072 (JP)
(74) Representative: Plasseraud IP
(86) International application number: PCT/JP2023/022630
(87) International publication number: WO 2023/248986

(57) **Abstract**

An embolization device (1) for a bump in a lumen, comprising a basket (10) for housing an embolic object, wherein the basket (10) has a plurality of wires (20), the basket (10) has a distal end side bundling portion (30) at which the plurality of wires (20) are bundled and fixed on a distal side (10d) of the basket (10), a proximal end side bundling portion (40) at which the plurality of wires (20) are bundled and fixed on a proximal side of the basket (10), and a housing portion (50) which is a portion between the distal end side bundling portion (30) and the proximal end side bundling portion (40) at the plurality of wires (20), a proximal end (30p) of the distal end side bundling portion (30) is located on the proximal side with respect to a distal end (50d) of the housing portion (50), and a distance (D1) between a distal end (30d) of the distal end side bundling portion (30) and the distal end (50d) of the housing portion (50) in a longitudinal direction of the basket (10) is 1/10 or more of a length (D2) from the distal end (50d) to a proximal end (50p) of the housing portion (50).

## Description

### TECHNICAL FIELD

The present invention relates to an embolization device for forming emboli at lesion sites of blood vessels.

### BACKGROUND ART

Endovascular treatment is one of the treatment methods for vascular lesions such as head and neck aneurysms, arteriovenous malformations, arteriovenous fistulas, pulmonary vascular malformations, renal vascular malformations, renal arteries, and abdominal aneurysms. As an endovascular treatment, for example, embolization, in which a basket-shaped device is indwelled at a blood vessel lesion site such as an aneurysm to modify blood flow and prevent rupture of the aneurysm, is known.

For example, Patent Literature 1 describes a device including a self-expanding resilient permeable shell having a proximal end, a distal end, and a longitudinal axis, including a plurality of resilient filaments with a woven structure, and having a radially constrained elongated state configured for delivery within a microcatheter and an expanded relaxed state configured to allow blood flow through openings at a velocity below a thrombotic threshold velocity. Patent Literature 2 describes a filament device including a self-expanding resilient permeable shell having a proximal end, a distal end, and a longitudinal axis, the permeable shell including a plurality of elongate resilient filaments with a woven structure secured relative to each other at the proximal end and the distal end and. Patent Literature 3 describes a left atrial appendage closure medical device including: a delivery catheter having a lumen extending therethrough; and a left atrial appendage closure implant including a proximal collar, a distal collar, and a monolithic support frame extending between the proximal collar and the distal collar, the monolithic support frame including a first bend extending from the proximal collar to a second bend, a first segment extending from the second bend to a third bend, a second segment extending from the third bend to a fourth bend, and a third segment extending from the fourth bend to the distal collar, wherein the monolithic support frame is actuatable from a first constrained position to a second flowering position to a third mid-deployment position to a fourth unconstrained position. Patent Literature 4 describes a device including an implant including woven braided mesh, wherein the implant has a proximal end with a hub, a distal end, a longitudinal axis extending from the proximal end to the distal end, a distal region, a proximal region, and a transition region that lies substantially perpendicular to the longitudinal axis of the implant and extends between the distal and proximal regions, and also has an expanded configuration when deployed, the expanded implant has a region of maximum diameter that extends from a proximal portion of the distal region through the transition region and to a distal portion of the proximal region, and the diameter of a pore within the proximal portion of the distal region is larger than those of all pores in the distal portion of the proximal region. Patent Literature 5 describes a device including a collapsible member arranged such that, when the device is deployed, the member bridges a neck of an aneurysm and is in contact with an inner wall. Patent Literature 6 describes a device including: a wire; a selectively detachable joint; and an expandable cage, wherein the joint couples the cage to the wire, the expandable cage is capable of assuming a collapsed and expanded configuration, and the expanded configuration is self-assumed by the cage when the cage is freed of confinement, whereby the cage expands for deployment within a body lumen.

### CITATION LIST

### PATENT LITERATURE

PATENT LITERATURE 1: JP 2011-519632 T
PATENT LITERATURE 2: JP 2015-198957 A
PATENT LITERATURE 3: JP 2015-534881 T
PATENT LITERATURE 4: JP 2017-511203 T
PATENT LITERATURE 5: JP 2001-518320 T
PATENT LITERATURE 6: US 5,916,235 A

### SUMMARY OF INVENTION

### TECHNICAL PROBLEM

In addition to embolization in which a basket-shaped device is indwelled at a lesion site of a blood vessel, there is also embolization in which an embolic object for embolus formation such as a coil is indwelled inside a bump to fill the bump and promote formation of an embolus, thereby preventing rupture of the bump. In embolization in which an embolic object is indwelled inside a bump, when the opening of an aneurysm or the like in a blood vessel wall is large, the embolic object indwelled inside the bump may come out of the bump. In order to prevent the embolic object indwelled inside a bump from falling out, an indwelling device to be placed in a bump or in a blood vessel near the opening of the bump may be used. In the present invention, a basket-shaped device is indwelled inside a bump, and an embolic object such as a coil is placed inside the device, thereby preventing the placed embolic object from falling out to a parent artery.

When an embolic object is to be placed inside a device having a shape similar to those of the devices of Patent Literatures 1 to 4, although this method is different from the methods of use of the devices of Patent Literatures 1 to 4, it is difficult to place the embolic object inside the device. Depending on the shape and state of a bump, the direction of insertion of the embolic object into the device, the type of the embolic object, etc., the embolic object placed inside the device is likely to have dense and sparse portions, causing a problem that it is difficult to sufficiently fill the inside of the bump with the embolic object, or when the inside of the device is excessively filled with the embolic object, a bundling portion at which filaments included in the device are bundled at a distal end portion of the device is likely to protrude to the outside of the device, causing a problem that there is a risk of the protruding bundling portion damaging a blood vessel wall, etc. In addition, in the device as in Patent Literatures 5 and 6, the effect of preventing the embolic object indwelled in the bump from falling out is not sufficient, so that there is a problem that the embolic object may come out of the bump depending on the shape of the bump, etc.

In addition, as for the conventional devices as in Patent Literatures 1 to 6, the inside of the device can be filled with the embolic object only up to a certain volume, and thus, normally, the device cannot be expanded or deformed very much. Bumps in lumens such as aneurysm have various sizes and shapes, and to sufficiently fill the inside of a bump with the embolic object, it is necessary to prepare a large number of different types of devices for the sizes and shapes of bumps, so that there is room for improvement.

The present invention has been made in view of the above circumstances, and an object of the present invention is to provide an embolization device that makes it easier to fill the entirety of an inside with an embolic object and is less likely to damage a blood vessel wall, etc.

### SOLUTION TO THE PROBLEMS

An embolization device according to an embodiment of the present invention that can solve the above problems is as follows.
[1] An embolization device for a bump in a lumen, comprising
   a basket for housing an embolic object, wherein
   the basket has a plurality of wires,
   the basket has a distal end side bundling portion at which the plurality of wires are bundled and fixed on a distal side of the basket, a proximal end side bundling portion at which the plurality of wires are bundled and fixed on a proximal side of the basket, and a housing portion which is a portion between the distal end side bundling portion and the proximal end side bundling portion at the plurality of wires,
   a proximal end of the distal end side bundling portion is located on the proximal side with respect to a distal end of the housing portion, and
   a distance between a distal end of the distal end side bundling portion and the distal end of the housing portion in a longitudinal direction of the basket is 1/10 or more of a length from the distal end to a proximal end of the housing portion.
[2] The embolization device according to [1], wherein
   the basket has an initial state where the embolic object is not housed inside the basket and a housed state where the embolic object is housed inside the basket in a certain volume or larger,
   a length from a distal end of the basket to a proximal end of the basket in the longitudinal direction of the basket is increased when the basket is brought from the initial state to the housed state, and
   a point of a distal end of the wire located on a most distal side in the initial state is moved to a radially outer side of the basket when the basket is brought from the initial state to the housed state.
[3] The embolization device according to [1] or [2], wherein
   the basket has an initial state where the embolic object is not housed inside the basket and a housed state where the embolic object is housed inside the basket in a certain volume or larger, and
   a distance between the distal end of the distal end side bundling portion and the distal end of the housing portion in the housed state is shorter than a distance between the distal end of the distal end side bundling portion and the distal end of the housing portion in the initial state.
[4] The embolization device according to any one of [1] to [3], wherein, in a housed state where the embolic object is housed inside the basket in a certain volume or larger, the distance between the distal end of the distal end side bundling portion and the distal end of the housing portion is 1/16 or more of a length from a distal end of the basket to a proximal end of the basket.
[5] The embolization device according to any one of [1] to [4], wherein the distal end side bundling portion is located inside the basket.
[6] The embolization device according to any one of [1] to [5], wherein the basket has a mesh-like wall surface in which the plurality of wires intersect each other.
[7] The embolization device according to any one of [1] to [6], further comprising an outer tube having a distal end and a proximal end, wherein
   the basket can be placed in an inner cavity of the outer tube, and
   the distal end of the distal end side bundling portion is located on the distal side with respect to the distal end of the housing portion in a state where the basket is housed inside the outer tube.
[8] The embolization device according to any one of [1] to [7], further comprising an outer tube having a distal end and a proximal end, wherein
   the basket can be placed in an inner cavity of the outer tube, and
   a proximal end of the proximal end side bundling portion is located on the proximal side with respect to the proximal end of the housing portion in a state where the basket is housed inside the outer tube.
[9] The embolization device according to any one of [1] to [8], wherein
   at least one of the wires of the basket contains a nickel-titanium alloy, and
   the basket is extendable in the longitudinal direction and a radial direction of the basket.
[10] The embolization device according to any one of [1] to [8], wherein an X-ray impermeable portion containing an X-ray impermeable material is provided at at least one of the distal end side bundling portion and the proximal end side bundling portion.
[11] The embolization device according to any one of [1] to [10], further comprising:
   a basket pusher placed on the proximal side with respect to a proximal end of the proximal end side bundling portion; and
   a connection member placed on the proximal side with respect to the proximal end of the proximal end side bundling portion and on the distal side with respect to a distal end of the basket pusher and connecting the basket and the basket pusher.
[12] The embolization device according to [11], wherein the connection member is severable.
[13] The embolization device according to any one of [1] to [12], further comprising an outer tube having a distal end and a proximal end, wherein
   the basket can be placed in an inner cavity of the outer tube,
   in a state where the basket is housed inside the outer tube, the distal end side bundling portion has a first end which is an end portion on a side farther from the proximal end side bundling portion, and a second end which is an end portion on a side closer to the proximal end side bundling portion, and
   in a state where the entire basket has come out of the outer tube and no external force is applied to the basket, a positional relationship in a distal-proximal direction between the first end and the second end in a state where the basket is housed inside the outer tube is inverted.

### ADVANTAGEOUS EFFECTS OF INVENTION

In the embolization device of the present invention, since the proximal end of the distal end side bundling portion of the basket is located on the proximal side with respect to the distal end of the housing portion and the distance between the distal end of the distal end side bundling portion and the distal end of the housing portion in the longitudinal direction of the basket is 1/10 or more of the length from the distal end to the proximal end of the housing portion, a protrusion formed by the distal end side bundling portion exists inside the basket. The protrusion acts like a core inside the basket, and blocks the movement of the embolic object placed inside the basket. As a result, when filling the inside of the basket with the embolic object, the embolic object is less likely to move together in the basket in a direction in which the embolic object easily moves, so that it becomes easier to fill the entirety of the inside of the basket with the embolic object. In addition, since the proximal end of the distal end side bundling portion of the basket is located on the proximal side with respect to the distal end of the housing portion, even if the embolic object sent into the inside of the basket comes into contact with the distal end side bundling portion and the wires located on the proximal side with respect to the proximal end of the distal end side bundling portion and the inside of the basket is excessively filled with the embolic object, the distal end side bundling portion is less likely to protrude to the outside of the basket. Therefore, the distal end side bundling portion of the basket can be less likely to damage other objects such as a blood vessel wall. Furthermore, when the embolic object of a certain volume or larger is housed inside the basket, the volume of the entire basket is expanded, so that it is possible to deal with aneurysms having a wide range of sizes and shapes using one type of embolization device.

### BRIEF DESCRIPTION OF DRAWINGS

[FIG. 1] FIG. 1 is a cross-sectional view, of an embolization device according to one embodiment of the present invention, parallel to a longitudinal direction thereof.
[FIG. 2] FIG. 2 shows a cross-sectional view, of the embolization device shown in FIG. 1, parallel to the longitudinal direction thereof in an initial state.
[FIG. 3] FIG. 3 shows a cross-sectional view, of the embolization device shown in FIG. 1, parallel to the longitudinal direction thereof in a housed state where an embolic object is housed up to a certain volume or larger.
[FIG. 4] FIG. 4 shows a cross-sectional view, of an embolization device according to another embodiment of the present invention, parallel to a longitudinal direction thereof in an initial state.
[FIG. 5] FIG. 5 shows a cross-sectional view, of the embolization device shown in FIG. 1, parallel to the longitudinal direction thereof in a state where a basket is housed inside an outer tube.
[FIG. 6] FIG. 6 shows a cross-sectional view, of the embolization device shown in FIG. 1, parallel to the longitudinal direction thereof in a state where the basket has come out of the outer tube and no external force is applied to the basket.
[FIG. 7] FIG. 7 shows a schematic diagram of a state where a conventional basket is indwelled in a bump and an embolic object is housed inside the basket.
[FIG. 8] FIG. 8 shows a schematic diagram of states where the embolization device shown in FIG. 1 is indwelled in a bump and the embolic object is housed inside the basket in a volume less than a certain volume and a volume equal to or larger than the certain volume.

### DESCRIPTION OF EMBODIMENTS

Hereinafter, the present invention is specifically described below based on the following embodiments; however, the present invention is not restricted by the embodiments described below of course, and can be certainly put into practice after appropriate modifications within in a range meeting the gist of the above and the below, all of which are included in the technical scope of the present invention. In the drawings, hatching or a reference sign for a member may be omitted for convenience, and in such a case, the description and other drawings should be referred to. In addition, sizes of various members in the drawings may differ from the actual sizes thereof, since priority is given to understanding the features of the present invention.

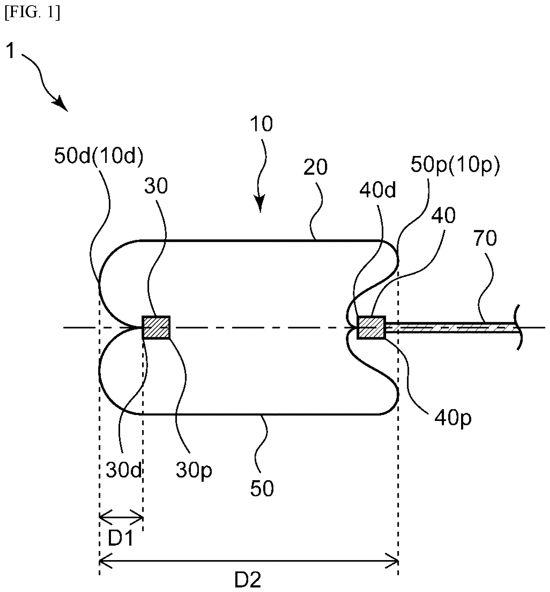

FIG. 1 is a cross-sectional view, of an embolization device 1 according to an embodiment of the present invention, parallel to a longitudinal direction thereof. In FIG. 1, for facilitating understanding of the positional relationship between a distal end side bundling portion 30, a proximal end side bundling portion 40, and a housing portion 50, only some of a plurality of wires 20 of a basket 10 are shown, and the other wires are not shown.

In the present invention, the proximal side refers to the hand side of a user with respect to a direction in which the embolization device 1 extends, and the distal side refers to a side opposite to the proximal side, that is, a side (lesion site side) on which treatment is performed with the embolization device 1. Also, the direction in which the embolization device 1 extends is referred to as a longitudinal direction. In other words, the longitudinal direction is a distal-proximal direction of the embolization device 1. The diameter direction of the basket 10 refers to the radial direction of the basket 10, a radially inward direction refers to a direction toward the axial center side of the basket 10, and a radially outward direction refers to a direction toward the side opposite to the inward direction. In FIG. 1, the right side of the drawing is the proximal side, and the left side of the drawing is the distal side.

As shown in FIG. 1, the embolization device 1 of the present invention is an embolization device 1 for a bump in a lumen, and has the basket 10 for housing an embolic object, the basket 10 has the plurality of wires 20, and has the distal end side bundling portion 30 at which the plurality of wires 20 are bundled and fixed on the distal side of the basket 10, the proximal end side bundling portion 40 at which the plurality of wires 20 are bundled and fixed on the proximal side of the basket 10, and the housing portion 50 which is a portion between the distal end side bundling portion 30 and the proximal end side bundling portion 40 at the plurality of wires 20, a proximal end 30p of the distal end side bundling portion 30 is located on the proximal side with respect to a distal end 50d of the housing portion 50, and a distance D1 between a distal end 30d of the distal end side bundling portion 30 and the distal end 50d of the housing portion 50 in the longitudinal direction of the basket 10 is 1/10 or more of a length D2 from the distal end 50d to a proximal end 50p of the housing portion 50.

The embolization device 1 is a device for embolizing a bump in a lumen. The embolization device 1 can be used for embolization, etc., in which the basket 10 for housing an embolic object is indwelled at a blood vessel lesion site such as an aneurysm to promote formation of an embolus, thereby preventing rupture of the aneurysm. The embolization device 1 introduces the basket 10 into a blood vessel and indwells the basket 10 at a target site. Specifically, the basket 10 is placed in a bump at a terminal portion or a side wall portion of a blood vessel or at a main tube peripheral portion or the like of a blood vessel, and the embolic object is placed inside the basket 10 placed in the bump or at the main tube peripheral portion or the like to promote formation of an embolus.

As shown in FIG. 1, the embolization device 1 has the basket 10. The basket 10 houses the embolic object therein.

Examples of the embolic object include long objects such as coils, wires, and string-like objects, granular objects, bag-like objects, etc. The embolic object may also be a solid, a semi-solid, a fluid, a gel-like material, a liquid, or the like. Examples of liquid embolic objects include liquids that harden, liquids that precipitate, etc. Among them, the embolic object is preferably a solid long object such as a coil. When the embolic object is a long object, it is easy to fill the inside of the basket 10 with the embolic object, so that embolization using the embolization device 1 can be efficiently performed.

As shown in the FIG. 1, the basket 10 has the plurality of wires 20. The basket 10 is preferably capable of expanding and contracting. Specifically, preferably, when the basket 10 receives an external force, the basket 10 is brought into a state where the outer shape thereof is squeezed and reduced in diameter, and when the basket 10 receives no external force, the basket 10 is brought into a state where the outer shape thereof is expanded and increased in diameter. When the basket 10 is placed in a bump, the basket 10 may come into contact with the wall surface of the bump and become deformed by an external force received therefrom.

The material forming the wires 20 preferably has elasticity, and examples thereof include metal wire materials that are single wires, flat wires, multiple wires, composite material wires, or twisted wires made of stainless steel such as SUS304 and SUS316, platinum, nickel, cobalt, chromium, titanium, tungsten, aluminum, gold, silver, nickel-titanium alloys, cobalt-chromium alloys, etc. Among them, the material forming the wires 20 is more preferably a metal wire material having superelasticity made of a nickel-titanium alloy or the like. When the material forming the wires 20 is a metal wire material, the elasticity of each wire 20 is enhanced, so that the amount of return to the original shape is large even if the deformation amount of the basket 10 is large. As a result, the shape of the basket 10 can be less likely to be lost.

It is sufficient that the number of wires 20 of the basket 10 is a plural number, and the number of wires 20 can be selected according to the inner diameter of an in-vivo lumen such as an aneurysm. In the drawings, the number of the wires 20 of the basket 10 is limited. However, in an embodiment of the present invention, the basket 10 can be, for example, a basket 10 including 8 to 64 wires 20. The wire diameter of each wire 20 can be set according to the size of the basket 10, the number and the material of the wires 20, etc. The number of wires 20 of the basket 10 is preferably 16 or larger and 32 or less.

As shown in FIG. 1, the basket 10 has the distal end side bundling portion 30 at which the plurality of wires 20 are bundled and fixed on the distal side of the basket 10, the proximal end side bundling portion 40 at which the plurality of wires 20 are bundled and fixed on the proximal side of the basket 10, and the housing portion 50 which is a portion between the distal end side bundling portion 30 and the proximal end side bundling portion 40 at the plurality of wires 20. The distal side of the basket 10 refers to a distal portion of the basket 10, and the proximal side of the basket 10 refers to a proximal portion of the basket 10. That is, the basket 10 has the distal end side bundling portion 30 at which the plurality of wires 20 are bundled and fixed, the proximal end side bundling portion 40 at which the plurality of wires 20 are bundled and fixed on the proximal side with respect to the distal end side bundling portion 30, and the housing portion 50 which is a portion between the distal end side bundling portion 30 and the proximal end side bundling portion 40 in a portion where the wires 20 extend.

Examples of a method for bundling and fixing the plurality of wires 20 at the distal end side bundling portion 30 and the proximal end side bundling portion 40 include welding the plurality of wires 20, crimping the plurality of wires 20 together using another member, adhering the plurality of wires 20 using an adhesive, and fixing the plurality of wires 20 with a brazing material. Among them, the plurality of wires 20 are preferably crimped and fixed using separate components at the distal end side bundling portion 30 and the proximal end side bundling portion 40. By crimping and fixing the plurality of wires 20 using separate components at the distal end side bundling portion 30 and the proximal end side bundling portion 40, the fixing strength of the plurality of wires 20 can be easily increased. As a result, the wires 20 bundled and fixed at the distal end side bundling portion 30 and the proximal end side bundling portion 40 are less likely to fall out, so that the basket 10 can be less likely to be damaged.

Examples of the separate components for bundling and fixing the plurality of wires 20 at the distal end side bundling portion 30 and the proximal end side bundling portion 40 include a ring-shaped member, a member obtained by forming a slit in a ring and having a C-shaped cross-section, a coil-like member obtained by winding a wire material, and a member for tying and fixing with a string-like object. Among them, each separate component for bundling and fixing the plurality of wires 20 is preferably a ring-shaped member, and the ring-shaped member is more preferably swaged. When each separate component for bundling and fixing the plurality of wires 20 is a ring-shaped member and is swaged, the plurality of wires 20 can be firmly fixed, and the plurality of wires 20 can be less likely to be de-bundled.

As the material forming each separate component for bundling and fixing the plurality of wires 20, for example, a material having less elasticity than the material forming the wires 20 of the basket 10 can be used. In particular, the material forming each separate component for bundling and fixing the plurality of wires 20 is preferably a platinum alloy such as Pt-W, Pt-Ir, or Pt-Pd. When the material forming each separate component for bundling and fixing the plurality of wires 20 is a platinum alloy, the fixing strength of the plurality of wires 20 can be increased, and the durability of each separate component for bundling and fixing the plurality of wires 20 can be improved.

The basket 10 is preferably formed in a cage shape in the housing portion 50, by braiding a plurality of bent wires 20, or right- and left-handed spiral wires 20, or the like. In particular, the housing portion 50 of the basket 10 is preferably formed by braiding right-handed spiral wires 20 and left-handed spiral wires 20. When the housing portion 50 of the basket 10 is formed by braiding right-handed spiral wires 20 and left-handed spiral wires 20, the basket 10 can have a cage shape having a mesh-like wall surface formed by the plurality of wires 20 intersecting each other. As a result, it becomes easier to send the embolic object into the inside of the basket 10 through the opening of the mesh-like wall surface of the basket 10, and the embolic object housed inside the basket 10 is less likely to come out of the basket 10, making it easier to efficiently promote formation of an embolus in a bump in a lumen.

As shown in FIG. 1, the proximal end 30p of the distal end side bundling portion 30 is located on the proximal side with respect to the distal end 50d of the housing portion 50, and the distance D1 between the distal end 30d of the distal end side bundling portion 30 and the distal end 50d of the housing portion 50 in the longitudinal direction of the basket 10 is 1/10 or more of the length D2 from the distal end 50d to the proximal end 50p of the housing portion 50.

When the proximal end 30p of the distal end side bundling portion 30 is located on the proximal side with respect to the distal end 50d of the housing portion 50 and the distance D1 between the distal end 30d of the distal end side bundling portion 30 and the distal end 50d of the housing portion 50 in the longitudinal direction of the basket 10 is 1/10 or more of the length D2 from the distal end 50d to the proximal end 50p of the housing portion 50, a protrusion formed by the distal end side bundling portion 30 exists inside the basket 10. The protrusion formed by the distal end side bundling portion 30 acts like a core inside the basket 10, and blocks the movement of the embolic object placed inside the basket 10, so that the embolic object is less likely to move. As a result, when filling the basket 10 with the embolic object, the embolic object is less likely to move together in the basket 10 in a direction in which the embolic object easily moves, so that it becomes easier to fill the entirety of the inside of the basket 10 with the embolic object. In addition, when the proximal end 30p of the distal end side bundling portion 30 is located on the proximal side with respect to the distal end 50d of the housing portion 50, even if the embolic object sent into the inside of the basket 10 comes into contact with the distal end side bundling portion 30 and the wires 20 located on the proximal side with respect to the proximal end 30p of the distal end side bundling portion 30, the distal end side bundling portion 30 is less likely to protrude to the outside of the basket 10. Therefore, the distal end side bundling portion 30 of the basket 10 is less likely to come into contact with other objects such as a blood vessel wall and can be less likely to damage the other objects.

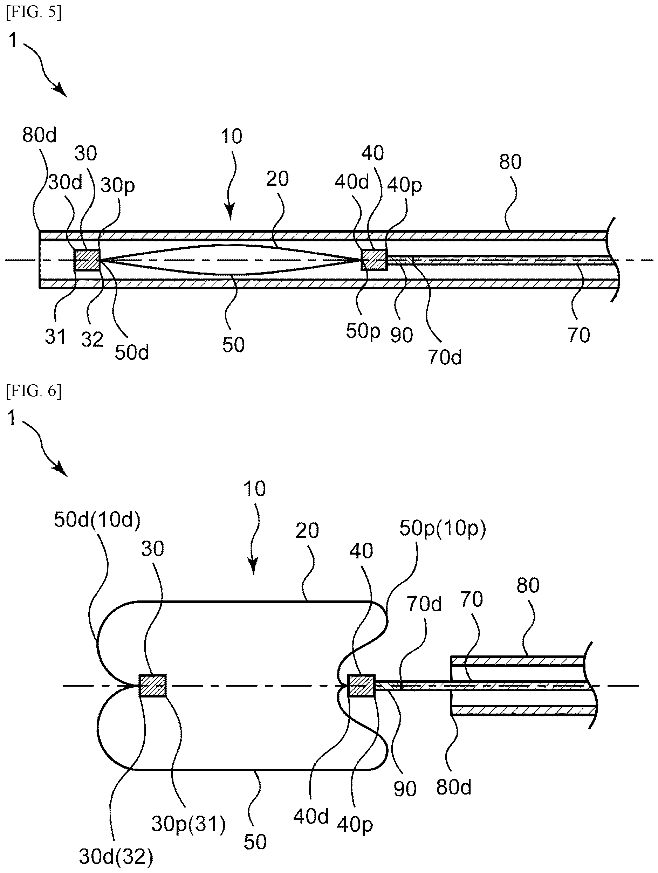

In the case where the embolization device 1 has an outer tube 80 having an inner cavity in which the basket 10 can be placed as shown in FIG. 6, the embolization device 1 of the present invention has a configuration in which, in a state where the basket 10 has come out of the outer tube 80 and receives no external force other than gravity, the proximal end 30p of the distal end side bundling portion 30 is located on the proximal side with respect to the distal end 50d of the housing portion 50 and the distance D1 between the distal end 30d of the distal end side bundling portion 30 and the distal end 50d of the housing portion 50 in the longitudinal direction of the basket 10 is 1/10 or more of the length D2 from the distal end 50d to the proximal end 50p of the housing portion 50. The outer tube 80 will be described in detail later.

The distance D1 between the distal end 30d of the distal end side bundling portion 30 and the distal end 50d of the housing portion 50 in the longitudinal direction of the basket 10 is 1/10 or more of the length D2 from the distal end 50d to the proximal end 50p of the housing portion 50, but is preferably 3/20 or more, more preferably 1/5 or more, further preferably 1/4 or more, even more preferably 3/10 or more, and particularly preferably 1/3 or more of the length D2. When the lower limit value of the ratio of the distance D1 to the length D2 is set within the above range, the length of the protrusion formed by the distal end side bundling portion 30 becomes sufficient, so that the effect of blocking the movement of the embolic object placed inside the basket 10 can be enhanced. In addition, the distance D1 between the distal end 30d of the distal end side bundling portion 30 and the distal end 50d of the housing portion 50 in the longitudinal direction of the basket 10 is preferably 15/16 or less, more preferably 9/10 or less, further preferably 4/5 or less, even more preferably 7/10 or less, and particularly preferably 2/3 or less of the length D2 from the distal end 50d to the proximal end 50p of the housing portion 50. When the upper limit value of the ratio of the distance D1 to the length D2 is set within the above range, it becomes easier to sufficiently ensure the internal space of the basket 10, so that it is possible to easily fill the inside of the basket 10 with the embolic object.

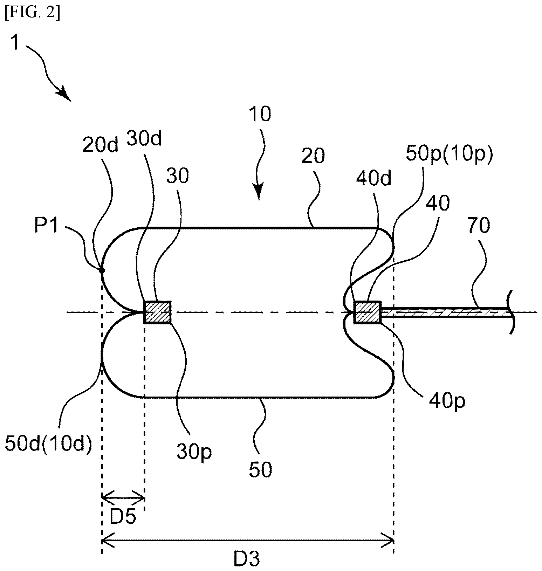

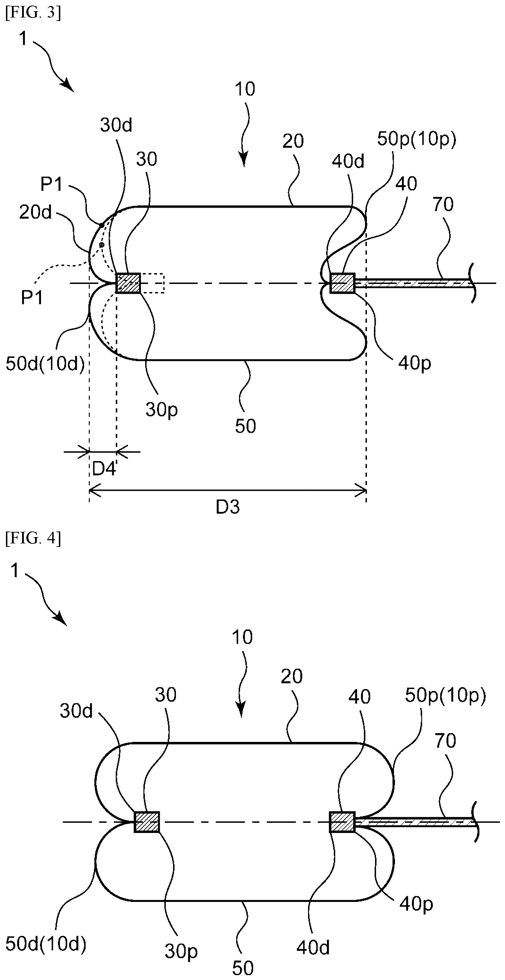

FIG. 2 is a cross-sectional view, of the embolization device 1, parallel to the longitudinal direction thereof in an initial state, FIG. 3 is a cross-sectional view, of the embolization device 1, parallel to the longitudinal direction thereof in a housed state, and FIG. 4 is a cross-sectional view, of an embolization device 1 according to another embodiment, parallel to a longitudinal direction thereof in an initial state. In FIG. 2 to FIG. 4 as well, only some of the plurality of wires 20 of the basket 10 are shown, and the other wires are not shown. In addition, in FIG. 2 to FIG. 4, the right side of the drawing is the proximal side, and the left side of the drawing is the distal side. In addition, in FIG. 3, for facilitating understanding of the positional relationship between each portion such as the distal end side bundling portion 30, the proximal end side bundling portion 40, and the housing portion 50, the embolic object housed inside the basket 10 is not shown.

It is preferable that the basket 10 has an initial state where the embolic object is not housed inside the basket 10 as shown in FIG. 2 and FIG. 4 and a housed state where the embolic object of a certain volume or larger is housed inside the basket 10 as shown in FIG. 3, a length D3 from a distal end 10d of the basket 10 to a proximal end 10p of the basket 10 in the longitudinal direction of the basket 10 is increased when the basket 10 is brought from the initial state to the housed state, and a point P1 of a distal end 20d of the wire 20 located on the most distal side in the initial state is moved to the radially outer side of the basket 10 when the basket 10 is brought from the initial state to the housed state. In FIG. 3, the embolization device 1 in the housed state is shown by a solid line, and the distal portion of the basket 10 in the initial state is shown by a broken line. When the basket 10 is brought from the initial state to the housed state, the protrusion formed by the distal end side bundling portion 30 is pushed outward toward the distal side of the basket 10 in the inside of the basket 10, so that the length D3 from the distal end 10d of the basket 10 to the proximal end 10p of the basket 10 in the longitudinal direction of the basket 10 is increased. In addition, when the basket 10 is brought from the initial state to the housed state, the wires 20 of the basket 10 are pushed and widened in the axial direction of the basket 10 by the embolic object placed inside the basket 10, and the point P1 of the distal end 20d of the wire 20 located on the most distal side in the initial state is moved to the radially outer side of the basket 10. That is, when the embolic object is housed inside the basket 10, the length D3 from the distal end 10d of the basket 10 to the proximal end 10p of the basket 10 in the longitudinal direction of the basket 10 is increased, and the point P1 of the distal end 20d of the wire 20 located on the most distal side in the initial state is moved to the radially outer side of the basket 10, so that the basket 10 is expanded in the longitudinal direction and the radial direction thereof. As a result, the basket 10 can be deformed to match the size, the shape, etc., of a bump for which embolization is performed, so that the embolization device 1 can be configured to deal with various bumps.

As for the embolic object of the certain volume or larger housed inside the basket 10 in the housed state, for example, in the case where the embolic object is a coil, the volume of the embolic object housed inside the basket 10 can be 25% or more, 30% or more, 35% or more, or 40% or more of the internal volume of the basket 10 in the initial state.

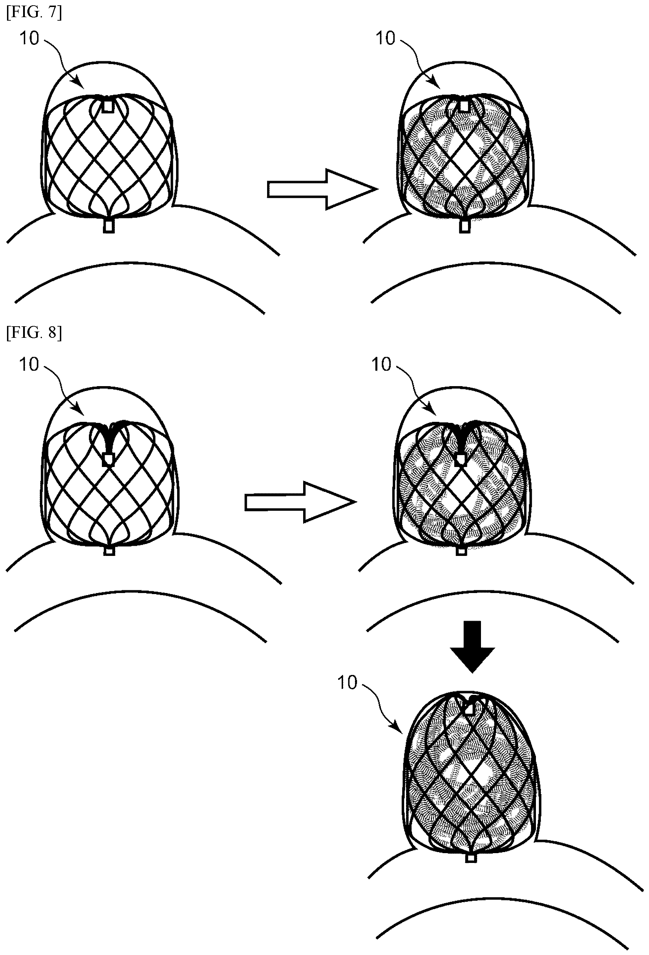

FIG. 7 is a schematic diagram of a state where a conventional basket 10 is indwelled in a bump and the embolic object is housed inside the basket 10, and FIG. 8 is a schematic diagram of a state where the embolization device of the present invention is indwelled in a bump and the embolic object is housed inside the basket 10. In FIG. 7, a drawing on the left side of an outline arrow shows an initial state where the basket 10 is placed in the bump, and a drawing on the right side of the outline arrow shows a state where the embolic object of the certain volume is housed inside the basket 10 placed in the bump. In FIG. 8, a drawing on the left side of an outline arrow shows an initial state where the basket 10 is placed in the bump, a drawing on the right side of the outline arrow shows a state where the inside of the basket 10 placed in the bump is filled with the embolic object of less than the certain volume, and a drawing on the lower side of a black arrow shows a housed state where the inside of the basket 10 placed in the bump is filled with the embolic object of the certain volume or larger. In addition, in FIG. 7 and FIG. 8, the lower side of the drawing is the proximal side, and the upper side of the drawing is the distal side.

As shown in FIG. 7, even if the inside of the conventional basket 10 is filled with the embolic object, the inside of the basket 10 can be filled with the embolic object only up to the certain volume. In particular, the basket 10 cannot be expanded very much in the axial direction of the basket 10, and the basket 10 cannot be deformed to a large extent. Therefore, as for the conventional basket 10, it is necessary to prepare a large number of baskets 10 for the sizes and shapes of bumps in order to deal with bumps having various sizes and shapes.

As shown in FIG. 8, in the embolization device 1 of the present invention, when the inside of the basket 10 is filled with the embolic object and further the inside of the basket 10 is filled with the embolic object of the certain volume or larger, the protrusion formed by the distal end side bundling portion is pushed outward toward the distal side of the basket 10 in the inside of the basket 10, so that the length from the distal end of the basket 10 to the proximal end of the basket 10 in the longitudinal direction of the basket 10 is increased. Therefore, it is possible for the basket 10 to expand in the axial direction thereof to be deformed to a large extent, so that it is possible to deal with bumps having various sizes and shapes using one type of embolization device.

As shown in FIG. 2 and FIG. 3, a distance D4 between the distal end 30d of the distal end side bundling portion 30 and the distal end 50d of the housing portion 50 in the housed state is preferably shorter than a distance D5 between the distal end 30d of the distal end side bundling portion 30 and the distal end 50d of the housing portion 50 in the initial state. When the distance D4 between the distal end 30d of the distal end side bundling portion 30 and the distal end 50d of the housing portion 50 in the housed state is shorter than the distance D5 between the distal end 30d of the distal end side bundling portion 30 and the distal end 50d of the housing portion 50 in the initial state, the length from the distal end 10d of the basket 10 to the proximal end 10p of the basket 10 is increased by the amount by which the distance between the distal end 30d of the distal end side bundling portion 30 and the distal end 50d of the housing portion 50 is decreased when the basket 10 is brought from the initial state to the housed state. Therefore, when the inside of the basket 10 is filled with the embolic object, the protrusion formed by the distal end side bundling portion 30 in the inside of the basket 10 is pushed out to the outside of the basket 10, so that it is possible to increase the entire length of the basket 10.

As shown in FIG. 3, in the housed state, the distance D4 between the distal end 30d of the distal end side bundling portion 30 and the distal end 50d of the housing portion 50 is preferably 1/16 or more of the length D3 from the distal end 10d of the basket 10 to the proximal end 10p of the basket 10. When the distance D4 between the distal end 30d of the distal end side bundling portion 30 and the distal end 50d of the housing portion 50 in the housed state is 1/16 or more of the length D3 from the distal end 10d of the basket 10 to the proximal end 10p of the basket 10, in a state where the inside of the basket 10 is filled with the embolic object, the distal end side bundling portion 30 of the basket 10 is less likely to protrude toward the distal side outside the basket 10, so that the distal end side bundling portion 30 is less likely to come into contact with other objects such as a blood vessel wall.

In the housed state, the distance D4 between the distal end 30d of the distal end side bundling portion 30 and the distal end 50d of the housing portion 50 is preferably 1/16 or more, more preferably 1/8 or more, and further preferably 1/5 or more of the length D3 from the distal end 10d of the basket 10 to the proximal end 10p of the basket 10. When the lower limit value of the ratio of the distance D4 to the length D3 in the housed state is set within the above range, the distal end side bundling portion 30 of the basket 10 is less likely to protrude toward the outside and the distal side of the basket 10. As a result, the distal end side bundling portion 30 is less likely to come into contact with other objects such as a blood vessel wall, so that the basket 10 can be less likely to damage other objects.

In the housed state, the distance D4 between the distal end 30d of the distal end side bundling portion 30 and the distal end 50d of the housing portion 50 is preferably 3/4 or less, more preferably 2/3 or less, and further preferably 1/2 or less of the length D3 from the distal end 10d of the basket 10 to the proximal end 10p of the basket 10. When the upper limit value of the ratio of the distance D4 to the length D3 in the housed state is set within the above range, it becomes easier to sufficiently ensure the internal space of the basket 10, so that the embolization device 1 can allow embolization to be effectively performed for a bump.

As shown in FIG. 1 to FIG. 3, the distal end side bundling portion 30 is preferably located inside the basket 10. When the distal end side bundling portion 30 is located inside the basket 10, the distal end side bundling portion 30 is less likely to be placed at the distal end of the basket 10. Therefore, even if the distal end of the basket 10 comes into contact with another object such as a blood vessel wall, the distal end side bundling portion 30 is less likely to come into contact with the other object, and the other object can be less likely to be damaged even if the basket 10 comes into contact with the other object.

The distal end 40d of the proximal end side bundling portion 40 may be located on the proximal side with respect to the proximal end 50p of the housing portion 50, but is preferably located on the distal side with respect to the proximal end 50p of the housing portion 50. When the distal end 40d of the proximal end side bundling portion 40 is located on the distal side with respect to the proximal end 50p of the housing portion 50, the proximal end side bundling portion 40 is less likely to protrude to the outside of the basket 10 at the proximal end 10p of the basket 10. Therefore, the proximal end side bundling portion 40 is less likely to come into contact with and damage other objects.

The basket 10 preferably has a mesh-like wall surface in which the plurality of wires 20 intersects each other. When the basket 10 has a mesh-like wall surface formed by the plurality of wires 20 intersecting each other, it can be easier to house the embolic object inside the basket 10 through the mesh-like opening where the plurality of wires 20 intersect each other. In addition, when the basket 10 has a mesh-like wall surface, since the opening of the wall surface of the basket 10 has a mesh-like shape in which the plurality of wires 20 intersect each other, the embolic object housed inside the basket 10 can be less likely to come out of the basket 10. As a result, it becomes easier to perform embolization for a bump using the embolization device 1.

FIG. 5 is a cross-sectional view, of the embolization device 1, parallel to the longitudinal direction thereof in a state where the basket 10 is housed inside the outer tube 80, and FIG. 6 is a cross-sectional view, of the embolization device 1, parallel to the longitudinal direction thereof in a state where the basket 10 has come out of the outer tube 80 and no external force is applied to the basket 10. In FIG. 5 and FIG. 6 as well, only some of the plurality of wires 20 of the basket 10 are shown, and the other wires are not shown. In addition, in FIG. 5 and FIG. 6, the right side of the drawing is the proximal side, and the left side of the drawing is the distal side.

As shown in FIG. 5 and FIG. 6, it is preferable that the outer tube 80 has a distal end 80d and a proximal end and that the basket 10 can be placed in the inner cavity of the outer tube 80. That is, it is preferable that the basket 10 is placed in a lumen of the outer tube 80. When the basket 10 can be placed in the inner cavity of the outer tube 80, the basket 10 can be transported to a target site in a state where the basket 10 is housed in the inner cavity of the outer tube 80. Therefore, the basket 10 is less likely to get caught on other objects such as a blood vessel wall, so that it is possible to smoothly transport the basket 10 to the target site.

It is preferable that the basket 10 is capable of expanding when coming out of the outer tube 80. That is, it is preferable that the basket 10 is placed in the inner cavity of the outer tube 80 and that the basket 10 is capable of expanding when released from the outer tube 80. It is preferable that, when the basket 10 is placed in the inner cavity of the outer tube 80, the basket 10 is in contact with the inner wall of the outer tube 80 and is brought into a state where the outer shape thereof is squeezed and reduced in diameter due to an external force received from the outer tube 80. In addition, it is preferable that, when the basket 10 is released from the outer tube 80, the basket 10 no longer receives the external force, and when no other external force is applied thereto, the basket 10 is brought into a state where the outer shape thereof is expanded and increased in diameter. When the basket 10 is capable of expanding when coming out of the outer tube 80, the basket 10 can have a smaller outer diameter in a state where the basket 10 is placed in the inner cavity of the outer tube 80. As a result, it becomes easier to transport the basket 10 to the target site, so that it becomes easier to perform procedures such as embolization using the embolization device 1.

The outer tube 80 is a tubular member extending in the longitudinal direction, and has at least one lumen extending in the longitudinal direction. The number of lumens of the outer tube 80 may be a plural number, but is preferably one. When the number of lumens of the outer tube 80 is one, the outer diameter of the outer tube 80 can be reduced, so that the less invasive nature of the embolization device 1 can be improved.

The material forming the outer tube 80 is preferably a resin or a metal.
Examples of the resin forming the outer tube 80 include polyamide-based resins, polyester-based resins, polyurethane-based resins, polyolefin-based resins, fluorine-based resins, vinyl chloride-based resins, silicone-based resins, natural rubber, etc. Only one of these materials may be used, or two or more of these materials may be used in combination. Among them, the resin forming the outer tube 80 is preferably at least one of polyamide-based resins, polyester-based resins, polyurethane-based resins, polyolefin-based resins, and fluorine-based resins. When the material forming the outer tube 80 is at least one of polyamide-based resins, polyester-based resins, polyurethane-based resins, polyolefin-based resins, and fluorine-based resins, the slipperiness of the surface of the outer tube 80 can be enhanced, and the insertability of the outer tube 80 into a lumen such as a blood vessel can be improved. A tube made of a resin and forming the outer tube 80 can be produced using a normal method such as extrusion molding and injection molding.

Examples of the metal forming the outer tube 80 include stainless steel such as SUS304 and SUS316, platinum, nickel, cobalt, chromium, titanium, tungsten, gold, nickel-titanium alloys, cobalt-chromium alloys, and combinations thereof. As a tube made of a metal and forming the outer tube 80, a tube obtained by spirally winding a metal wire material, a tube obtained by knitting a metal wire material, or the like may be used. In addition, the outer tube 80 may be a tube obtained by combining a metal and a resin. A tubular member formed from a resin and having a reinforcing material such as a metal wire material provided therein may be used as the outer tube 80. In the case where a tube-like member made of a resin and having a wire material provided therein is used as the outer tube 80, the wire material is preferably formed from a nickel-titanium alloy since the wire material has excellent shape memory and high elasticity. In addition, the wire material provided in the tubular member made of a resin may be the above-described metal, or a fiber material such as polyarylate fiber, aramid fiber, ultra-high molecular weight polyethylene fiber, PBO fiber, and carbon fiber. The fiber material forming the wire material may be monofilament or may be multifilament.

The outer tube 80 may be composed of a single layer or may be composed of a plurality of layers. In addition, in the longitudinal direction, a part of the outer tube 80 may be composed of a single layer, and the other part of the outer tube 80 may be composed of a plurality of layers.

The outer tube 80 is preferably coated with a hydrophilic resin on the outer surface of the outer tube 80. That is, the outer tube 80 preferably has a hydrophilic resin layer on the outer side of the outer tube 80. When the outer surface of the outer tube 80 is coated with a hydrophilic resin, the slipperiness of the outer tube 80 can be enhanced, so that the insertability thereof in an in-vivo lumen can be enhanced.

In addition, the outer tube 80 is preferably coated with a fluorine-based resin on the inner surface of the outer tube 80. That is, the outer tube 80 preferably has a fluorine-based resin layer on the inner side of the outer tube 80. When the outer tube 80 has a fluorine-based resin layer on the inner surface of the outer tube 80, the slipperiness of the inner surface of the outer tube 80 is improved, which makes it easier to move the basket 10 in the longitudinal direction in the inner cavity of the outer tube 80.

The cross-sectional shape of the outer tube 80 in a cross-section perpendicular to the longitudinal direction may be a circular shape, an elliptical shape, a polygonal shape, or a combination thereof. In addition, the cross-sectional shape of the lumen of the outer tube 80 in the cross-section perpendicular to the longitudinal direction may also be a circular shape, an elliptical shape, a polygonal shape, or a combination thereof.

The basket 10 is preferably deformable and slidable inside a tube having an inner diameter of 0.021 inch (0.5334 mm) or smaller, and is more preferably slidable inside a tube having an inner diameter of 0.017 inch (0.4318 mm) or smaller. In the case where the number of lumens of the outer tube 80 is one, it is preferable that the inner diameter of the outer tube 80 is 0.017 inch or smaller and the basket 10 is placed in the inner cavity of the outer tube 80. In addition, in the case where the outer tube 80 has a plurality of lumens, it is preferable that the inner diameter of the lumen in which the basket 10 is placed is 0.017 inch or smaller. When the basket 10 is deformable and is slidable inside a tube having an inner diameter of 0.017 inch or smaller, the outer diameter of the outer tube 80 can be reduced, so that the embolization device 1 can have good insertability and be less invasive.

As shown in FIG. 5, in a state where the basket 10 is housed inside the outer tube 80, the distal end 30d of the distal end side bundling portion 30 is preferably located on the distal side with respect to the distal end 50d of the housing portion 50. That is, it is preferable that, in a state where the entire basket 10 has come out of the outer tube 80 and no external force is applied to the basket 10, the proximal end 30p of the distal end side bundling portion 30 is located on the proximal side with respect to the distal end 50d of the housing portion 50, and in a state where the basket 10 is housed inside the outer tube 80, the distal end 30d of the distal end side bundling portion 30 is located on the distal side with respect to the distal end 50d of the housing portion 50. When the distal end 30d of the distal end side bundling portion 30 is located on the distal side with respect to the distal end 50d of the housing portion 50 in a state where the basket 10 is housed inside the outer tube 80, the wires 20 are less likely to be placed radially outward of the distal end side bundling portion 30 inside the outer tube 80. Therefore, the outer diameter of the basket 10 placed in the inner cavity of the outer tube 80 can be reduced, and the outer diameter of the outer tube 80 can also be reduced accordingly, so that the embolization device 1 can be less invasive.

As shown in FIG. 5, in a state where the basket 10 is housed inside the outer tube 80, the proximal end 40p of the proximal end side bundling portion 40 is preferably located on the proximal side with respect to the proximal end 50p of the housing portion 50. In addition, as shown in FIG. 6, in a state where the entire basket 10 has come out of the outer tube 80 and no external force is applied to the basket 10, the distal end 40d of the proximal end side bundling portion 40 is preferably located on the distal side with respect to the proximal end 50p of the housing portion 50. When the proximal end 40p of the proximal end side bundling portion 40 is located on the proximal side with respect to the proximal end 50p of the housing portion 50 in a state where the basket 10 is housed inside the outer tube 80, the wires 20 are less likely to be placed radially outward of the proximal end side bundling portion 40 inside the outer tube 80. As a result, the outer diameter of the basket 10 placed in the inner cavity of the outer tube 80 can be reduced, and the outer diameter of the outer tube 80 can also be reduced, so that the less invasive nature of the embolization device 1 can be improved.

The distal end 40d of the proximal end side bundling portion 40 may be located on the distal side with respect to the proximal end 50p of the housing portion 50, or may be located on the proximal side with respect to the proximal end 50p of the housing portion 50. In particular, it is preferable that the distal end 40d of the proximal end side bundling portion 40 is located on the distal side with respect to the proximal end 50p of the housing portion 50 as shown in FIG. 1 to FIG. 4. When the distal end 40d of the proximal end side bundling portion 40 is located on the distal side with respect to the proximal end 50p of the housing portion 50, the proximal end side bundling portion 40 is less likely to protrude at the proximal end 10p of the basket 10. Therefore, for example, when indwelling the basket 10 at a target site, even if the proximal end 10p of the basket 10 comes into contact with another object such as a blood vessel wall, the proximal end side bundling portion 40 can be prevented from coming into contact with and damaging the other object.

As shown in FIG. 1 to FIG. 4 and FIG. 6, the proximal end 40p of the proximal end side bundling portion 40 is preferably located on the distal side with respect to the proximal end 50p of the housing portion 50. When the proximal end 40p of the proximal end side bundling portion 40 is located on the distal side with respect to the proximal end 50p of the housing portion 50, the proximal end side bundling portion 40 does not exist at the proximal end 10p of the basket 10. As a result, when the proximal end 10p of the basket 10 comes into contact with another object, the proximal end side bundling portion 40 is less likely to come into contact with the other object, so that it becomes easier to prevent damaging the other object due to contact with the proximal end side bundling portion 40.

As for the configuration in which the distal end 40d of the proximal end side bundling portion 40 is located on the distal side with respect to the proximal end 50p of the housing portion 50, as shown in FIG. 1, the wires 20 may exist on the distal side with respect to the distal end 40d of the proximal end side bundling portion 40, and the proximal portion of the basket 10 may have a recess shape, or, as shown in FIG. 4, the wires 20 may exist on the proximal side with respect to a proximal end 40p of the proximal end side bundling portion 40, and at least a part of the proximal end side bundling portion 40 may be located inside the basket 10. That is, the proximal end side bundling portion 40 may be located outside the basket 10, or may be located inside the basket 10.

It is preferable that at least one wire 20 of the basket 10 contains a nickel-titanium alloy and the basket 10 is extendable in the longitudinal direction and the radial direction of the basket 10. When at least one wire 20 of the basket 10 contains a nickel-titanium alloy, the wire 20 becomes highly elastic, allowing the basket 10 to be deformed to a large extent. In addition, when the basket 10 is extendable in the longitudinal direction and the radial direction of the basket 10, it is possible to increase the volume of the basket 10. That is, when at least one wire 20 of the basket 10 contains a nickel-titanium alloy and the basket 10 is extendable in the longitudinal direction and the radial direction of the basket 10, the basket 10 can be expanded to match the size of a bump in a lumen, so that the basket 10 can be brought into close contact with the wall surface of the bump. As a result, it becomes easier to perform embolization for the bump.

It is preferable that an X-ray impermeable portion containing an X-ray impermeable material is provided at at least one of the distal end side bundling portion 30 and the proximal end side bundling portion 40. When the X-ray impermeable portion is provided at at least one of the distal end side bundling portion 30 and the proximal end side bundling portion 40, the position of at least one of the distal end side bundling portion 30 and the proximal end side bundling portion 40 at which the X-ray impermeable portion is provided can be confirmed under X-ray fluoroscopy, so that it is possible to grasp the position of the basket 10 in a body.

Examples of the X-ray impermeable material include platinum, gold, tungsten, iridium, palladium, tantalum, and alloys each obtained by combining at least one of these, etc.

As the X-ray impermeable portion provided at at least one of the distal end side bundling portion 30 and the proximal end side bundling portion 40, the above-described material forming each separate component for bundling and fixing the plurality of wires 20 may contain the X-ray impermeable material, and each separate component for bundling and fixing the plurality of wires 20 may also serve as the X-ray impermeable portion. When each separate component for bundling and fixing the plurality of wires 20 also serves as the X-ray impermeable portion, the number of components constituting the basket 10 can be prevented from being increased, so that it is possible to increase the production efficiency of the embolization device 1.

At least one wire 20 of the basket 10 may contain the X-ray impermeable material. When at least one wire 20 contains the X-ray impermeable material, the state of expansion of the basket 10 and the position of the basket 10 can be confirmed under X-ray fluoroscopy, so that it becomes easier to smoothly perform procedures using the embolization device 1.

At least one wire 20 of the basket 10 may have a configuration of having a core portion and an outer layer covering the core portion. When at least one wire 20 has a configuration of having a core portion and an outer layer, the material forming the core portion and the material forming the outer layer can be different, so that it is possible to give various characteristics to the wire 20.

In the case where at least one wire 20 of the basket 10 has a configuration of having a core portion and an outer layer, examples of the materials forming the core portion and the outer layer include stainless steel such as SUS304 and SUS316, and metals such as platinum, nickel, cobalt, chromium, titanium, tungsten, aluminum, gold, silver, lead, barium, iodine, iridium, stainless steel, titanium, nickel-titanium alloys, and cobalt-chromium alloys. Among them, at least one wire 20 preferably has a configuration of having a core portion containing the X-ray impermeable material and an outer layer portion containing a nickel-titanium alloy. When at least one wire 20 has a configuration of having a core portion containing the X-ray impermeable material and an outer layer portion containing a nickel-titanium alloy, it is possible to enhance the elasticity of the basket 10 by the nickel-titanium alloy of the outer layer portion to make it easier to expand the basket 10, while enhancing the visibility of the basket 10 under X-ray fluoroscopy by the X-ray impermeable material of the core portion.

As shown in FIG. 1 to FIG. 6, the embolization device 1 preferably has a basket pusher 70 placed on the proximal side with respect to the proximal end 40p of the proximal end side bundling portion 40. The basket pusher 70 is connected directly or indirectly to the basket 10, and the basket 10 can be moved by pushing and pulling the basket pusher 70 with respect to the hand side.

The material forming the basket pusher 70 is preferably a metal, and examples thereof include metals such as stainless steel, carbon steel, and nickel-titanium alloys. Among them, the material forming the basket pusher 70 is preferably stainless steel. When the material forming the basket pusher 70 is stainless steel, the rigidity of the basket pusher 70 can be increased. As a result, the force applied to the basket pusher 70 can be efficiently transmitted to the basket 10, so that it becomes easier to perform the operation of moving the basket 10 in the longitudinal direction.

Although not shown, the basket pusher 70 may include, at a proximal end portion thereof, a handle for controlling the position in the longitudinal direction or rotation thereof. When the basket pusher 70 has the handle, it becomes easier to perform the operation of pushing and pulling the basket pusher 70.

As shown in FIG. 5 and FIG. 6, the embolization device 1 preferably further has a connection member 90 which is placed on the proximal side with respect to the proximal end 40p of the proximal end side bundling portion 40 and on the distal side with respect to a distal end 70d of the basket pusher 70 and connects the basket 10 and the basket pusher 70. When the embolization device 1 has the connection member 90 which connects the basket 10 and the basket pusher 70, it is possible to easily connect the basket 10 and the basket pusher 70.

It is preferable that the connection member 90 is severable. That is, it is preferable that it is possible to detach the basket 10 from the basket pusher 70 by severing the connection member 90. When the connection member 90 is severable, the basket 10 can be detached from the basket pusher 70 by severing the connection member 90 after the basket 10 is transported to a target site. Therefore, it is possible to easily indwell the basket 10 at the target site.

As a method for severing the connection member 90, various methods using a mechanical severing mechanism, thermal cutting, thermal, electrical, and chemical severing, etc., can be used. Examples of the connection member 90 include rod-like objects, string-like objects, clips, members integrated by fitting a recess and a projection or the like to each other, etc. As the material forming the connection member 90, synthetic resins, metals, etc., can be used. The connection member 90 may be a member different from the basket 10 or the basket pusher 70, or may be a part of the basket 10 or the basket pusher 70.

It is preferable that the material forming the connection member 90 has a property of melting due to heat and the embolization device 1 has a heating mechanism for heating the connection member 90. When the material forming the connection member 90 has a property of melting due to heat and the embolization device 1 has the heating mechanism for heating the connection member 90, the connection member 90 can be melted and broken by the heating mechanism heating the connection member 90, so that the basket 10 can be detached from the basket pusher 70. Therefore, the basket 10 and the basket pusher 70 can be firmly connected by the connection member 90 until the heating mechanism is activated, and the connection member 90 can be easily severed when the heating mechanism is activated. As a result, it is possible to reliably and easily indwell the basket 10 at a target site.

As the material forming the connection member 90 and having a property of melting due to heat, thermoplastic resins are preferable, and among them, polyvinyl alcohol (PVA) is more preferable. When the material forming the connection member 90 is PVA, the connection member 90 is easily severed, so that the embolization device 1 can be easily handled.

Although not shown, it is preferable that the heating mechanism is connected to the basket pusher 70. When the heating mechanism is connected to the basket pusher 70, the connection member 90 can be heated via the basket pusher 70. Therefore, it is not necessary to additionally provide a member for transmitting the heat of the heating mechanism to the connection member 90 and the size of the embolization device 1 can be reduced. The heating mechanism may be directly connected to the basket pusher 70, or may be indirectly connected to the basket pusher 70 via another member.

It is preferable that, as shown in FIG. 5, in a state where the basket 10 is housed inside the outer tube 80, the distal end side bundling portion 30 has a first end 31 which is an end portion on the side farther from the proximal end side bundling portion 40, and a second end 32 which is an end portion on the side closer to the proximal end side bundling portion 40, and as shown in FIG. 6, in a state where the entire basket 10 has come out of the outer tube 80 and no external force is applied to the basket 10, the positional relationship in the distal-proximal direction between the first end 31 and the second end 32 in a state where the basket 10 is housed inside the outer tube 80 is inverted. That is, it is preferable that, in a state where the entire basket 10 has come out of the outer tube 80 and no external force is applied to the basket 10, the second end 32 is located on the distal side with respect to the first end 31. Specifically, it is preferable that the positional relationship between the first end 31 and the second end 32 of the distal end side bundling portion 30 in the distal-proximal direction is inverted between the case where the basket 10 is housed inside the outer tube 80 and the case where the entire basket 10 has come out of the outer tube 80 and no external force is applied to the basket 10. In other words, it is preferable that the positional relationship between the first end 31 and the second end 32 of the distal end side bundling portion 30 in the distal-proximal direction is inverted between a state where the basket 10 is housed inside the outer tube 80 and a state where the entire basket 10 has come out of the outer tube 80 and no external force is applied to the basket 10.

When the first end 31 is located on the distal side with respect to the second end 32 in a state where the basket 10 is housed inside the outer tube 80, the positions of the wires 20 included in the basket 10 and the distal end side bundling portion 30 do not overlap each other inside the outer tube 80. Therefore, the diameter of the basket 10 is less likely to be increased, and the outer tube 80 having a smaller diameter can be used, so that the embolization device 1 can be less invasive. In addition, when the second end 32 is located on the distal side with respect to the first end 31 in a state where the entire basket 10 has come out of the outer tube 80 and no external force is applied to the basket 10, the wires 20 located at the distal end portion of the housing portion 50 are bent. As a result, even if the distal end of the basket 10 comes into contact with another object such as a blood vessel wall, the distal end can be less likely to damage the other object.

As described above, the embolization device of the present invention is an embolization device for a bump in a lumen, including a basket for housing an embolic object, wherein: the basket has a plurality of wires; the basket has a distal end side bundling portion at which the plurality of wires are bundled and fixed on a distal side of the basket, a proximal end side bundling portion at which the plurality of wires are bundled and fixed on a proximal side of the basket, and a housing portion which is a portion between the distal end side bundling portion and the proximal end side bundling portion at the plurality of wires; a proximal end of the distal end side bundling portion is located on the proximal side with respect to a distal end of the housing portion; and a distance between a distal end of the distal end side bundling portion and the distal end of the housing portion in a longitudinal direction of the basket is 1/10 or more of a length from the distal end to a proximal end of the housing portion. Since the embolization device of the present invention is configured as described above, a protrusion formed by the distal end side bundling portion exists inside the basket. The protrusion formed by the distal end side bundling portion acts like a core inside the basket, and blocks the movement of the embolic object placed inside the basket, so that the embolic object is less likely to move. As a result, when filling the basket with the embolic object, the entire embolic object is less likely to move together in the basket in a direction in which the embolic object easily moves, so that it becomes easier to fill the entirety of the inside of the basket with the embolic object. In addition, since the proximal end of the distal end side bundling portion is located on the proximal side with respect to the distal end of the housing portion, even if the embolic object sent into the inside of the basket comes into contact with the distal end side bundling portion and the wires located on the proximal side with respect to the proximal end of the distal end side bundling portion, the distal end side bundling portion is less likely to protrude to the outside of the basket, so that other objects such as a blood vessel wall can be less likely to be damaged. Furthermore, when the embolic object of a certain volume or larger is housed inside the basket, the volume of the entire basket is expanded, so that it is possible to deal with aneurysms having a wide range of sizes and shapes using one type of embolization device.

This application claims priority to Japanese Patent Application No. 2022-100663, filed on June 22, 2022. All of the contents of the Japanese Patent Application No. 2022-100663, filed on June 22, 2022, are incorporated by reference herein.

### REFERENCE SIGNS LIST

1: embolization device
10: basket
10d: distal end of the basket
10p: proximal end of the basket
20: wire
20d: distal end of the wire
30: distal end side bundling portion
30d: distal end of the distal end side bundling portion
30p: a proximal end of the distal end side bundling portion
31: first end
32: second end
40: proximal end side bundling portion
40d: distal end of the proximal end side bundling portion
40p: proximal end of the proximal end side bundling portion
50: housing portion
50d: distal end of the housing portion
50p: proximal end of the housing portion
70: basket pusher
70d: distal end of the basket pusher
80: outer tube
80d: distal end of the outer tube
90: connection member
P1: point of a distal end of the wire located on the most distal side in the initial state
D1: distance between a distal end of the distal end side bundling portion and the distal end of the housing portion in the longitudinal direction of the basket
D2: length from the distal end to a proximal end of the housing portion
D3: length from a distal end of the basket to a proximal end of the basket in the longitudinal direction of the basket
D4: distance between the distal end of the distal end side bundling portion and the distal end of the housing portion in the housed state
D5: distance between the distal end of the distal end side bundling portion and the distal end of the housing portion in the initial state

## Claims

1. An embolization device for a bump in a lumen, comprising
a basket for housing an embolic object, wherein
the basket has a plurality of wires,
the basket has a distal end side bundling portion at which the plurality of wires are bundled and fixed on a distal side of the basket, a proximal end side bundling portion at which the plurality of wires are bundled and fixed on a proximal side of the basket, and a housing portion which is a portion between the distal end side bundling portion and the proximal end side bundling portion at the plurality of wires,
a proximal end of the distal end side bundling portion is located on the proximal side with respect to a distal end of the housing portion, and
a distance between a distal end of the distal end side bundling portion and the distal end of the housing portion in a longitudinal direction of the basket is 1/10 or more of a length from the distal end to a proximal end of the housing portion.

2. The embolization device according to claim 1, wherein
the basket has an initial state where the embolic object is not housed inside the basket and a housed state where the embolic object is housed inside the basket in a certain volume or larger,
a length from a distal end of the basket to a proximal end of the basket in the longitudinal direction of the basket is increased when the basket is brought from the initial state to the housed state, and
a point of a distal end of the wire located on a most distal side in the initial state is moved to a radially outer side of the basket when the basket is brought from the initial state to the housed state.

3. The embolization device according to claim 1, wherein
the basket has an initial state where the embolic object is not housed inside the basket and a housed state where the embolic object is housed inside the basket in a certain volume or larger, and
a distance between the distal end of the distal end side bundling portion and the distal end of the housing portion in the housed state is shorter than a distance between the distal end of the distal end side bundling portion and the distal end of the housing portion in the initial state.

4. The embolization device according to claim 1, wherein, in a housed state where the embolic object is housed inside the basket in a certain volume or larger, the distance between the distal end of the distal end side bundling portion and the distal end of the housing portion is 1/16 or more of a length from a distal end of the basket to a proximal end of the basket.

5. The embolization device according to claim 1, wherein the distal end side bundling portion is located inside the basket.

6. The embolization device according to claim 1, wherein the basket has a mesh-like wall surface in which the plurality of wires intersect each other.

7. The embolization device according to claim 1, further comprising an outer tube having a distal end and a proximal end, wherein
the basket can be placed in an inner cavity of the outer tube, and
the distal end of the distal end side bundling portion is located on the distal side with respect to the distal end of the housing portion in a state where the basket is housed inside the outer tube.

8. The embolization device according to claim 1, further comprising an outer tube having a distal end and a proximal end, wherein
the basket can be placed in an inner cavity of the outer tube, and
a proximal end of the proximal end side bundling portion is located on the proximal side with respect to the proximal end of the housing portion in a state where the basket is housed inside the outer tube.

9. The embolization device according to claim 1, wherein
at least one of the wires of the basket contains a nickel-titanium alloy, and
the basket is extendable in the longitudinal direction and a radial direction of the basket.

10. The embolization device according to claim 1, wherein an X-ray impermeable portion containing an X-ray impermeable material is provided at at least one of the distal end side bundling portion and the proximal end side bundling portion.

11. The embolization device according to claim 1, further comprising:
a basket pusher placed on the proximal side with respect to a proximal end of the proximal end side bundling portion; and
a connection member placed on the proximal side with respect to the proximal end of the proximal end side bundling portion and on the distal side with respect to a distal end of the basket pusher and connecting the basket and the basket pusher.

12. The embolization device according to claim 11, wherein the connection member is severable.

13. The embolization device according to claim 1, further comprising an outer tube having a distal end and a proximal end, wherein
the basket can be placed in an inner cavity of the outer tube,
in a state where the basket is housed inside the outer tube, the distal end side bundling portion has a first end which is an end portion on a side farther from the proximal end side bundling portion, and a second end which is an end portion on a side closer to the proximal end side bundling portion, and
in a state where the entire basket has come out of the outer tube and no external force is applied to the basket, a positional relationship in a distal-proximal direction between the first end and the second end in a state where the basket is housed inside the outer tube is inverted.
